# EUROPEAN PATENT SPECIFICATION

(11) **EP 2 552 367 B1**
(45) Date of publication and mention of the grant of the patent: **02.07.2014**
(21) Application number: 11716028.3
(22) Date of filing: 15.03.2011
(51) Int. Cl.: A61F 9/00, B65D 83/00

(54) **DEVICE FOR THE ADMINISTRATION OF FLUID PRODUCTS**
VORRICHTUNG ZUR VERABREICHUNG VON FLÜSSIGPRODUKTEN
DISPOSITIF D'ADMINISTRATION DE PRODUITS FLUIDES

(30) Priority: 01.04.2010 IT MO20100094
(43) Date of publication of application: 06.02.2013
(73) Proprietor: Lameplast S.P.A., Frazione Rovereto Sul Secchia (IT)
(72) Inventor: FONTANA, Antonio, 41012 Carpi (MO) (IT)
(74) Representative: Brunacci, Marco
(86) International application number: PCT/IB2011/000537
(87) International publication number: WO 2011/121405

(56) References cited:
- WO-A1-2008/150115
- WO-A1-2010/075119
- WO-A2-2008/103649
- DE-A1- 3 202 275
- DE-A1- 3 305 898
- DE-A1- 4 423 261
- DE-C- 207 370
- DE-U1-202006 010 907
- DE-U1-202006 010 907
- FR-A1- 2 510 071
- FR-A1- 2 903 092
- US-A- 2 329 917
- US-A1- 2009 230 152
- US-B1- 6 364 163

## Description

### Technical Field

The present invention relates to a device for the administration of fluid products.

### Background Art

Different hygienic-sanitary devices are known for the administration of fluid products, such as liquids, pastes, gel or the like.

One of these devices is composed of traditional bottles having an open extremity for dispensing the product and made of a plastic material soft enough to allow crushing the walls of the bottle to push the fluid product towards the open extremity.

The shape of the deformable bottle can be cylindrical, round, oval, etc., or can be bellows-shaped, in which case the product containment bag consists of a receptacle that can be folded on itself.

The deformable bottles of traditional type are usually crushed by the user by means of the action of the fingers directed transversally to the fluid product outlet direction, and are therefore particularly practical and easy to use for example for the application of eye-drops or the like.

All these bottles do however have numerous drawbacks, such as, e.g., the fact that they are affected by inconvenient use restrictions.

In this respect, the fact is underlined that the dimensions and the conformation of these devices do not always allow dispensing the totality of the product contained in them, which, on the contrary, remains trapped at least in part in the bottle, and represents a pointless waste.

Other devices for the application of fluid products are furthermore known which consist of a cylindrical body suitable for containing the product and inside which a piston is sliding integral with the extremity of a push rod to operate like a traditional dispensing syringe.

The piston is usually composed of a flat plate inserted sliding on the inner side walls of the cylindrical body.

The cylindrical body has an open extremity through which the fluid product is dispensed while the push rod appears from the opposite extremity to operate the piston.

Dispensing of the fluid product is by means of the manual operation on the push rod in the direction of piston sliding towards the open extremity of the cylindrical body.

These syringe devices also have a number of drawbacks however, including the fact that they are often not easy to handle and practical to use.

In this respect in fact, it is underlined that while the deformable bottles of traditional type are usually crushed transversally to the direction of fluid product dispensing, the syringe devices instead require the application of an axial thrust by the user.

In particular uses, e.g., for the application of eye-drops or the like, therefore, such devices are very inconvenient to use taking into account the difficulty for the user to handle the cylindrical body and, at the same time, operate the push rod in axial direction.

It should not be forgotten, furthermore, that the syringe devices of traditional type are sometimes very complex to make and therefore have rather high production costs that negatively affect the final retail price, with the risk of making the products less appealing to consumers.

Other types of devices for delivering fluids are disclosed in the patent documents DE 207 370 (disclosing the preamble features of claim 1), US 2,329,917 and WO 2010/075119.

### Description of the Invention

The main object of the present invention is to provide a device for the administration of fluid products that has compact overall dimensions and is easy to handle, simple and quick to use by users, quick to manufacture, pack and package.

Another object of the present invention is to provide a device for the administration of fluid products that allows overcoming the mentioned drawbacks of the background art within the ambit of a simple, rational, easy to use and low cost solution.

The above objects are achieved by the present device for the administration of fluid products having the features of claim 1.

### Brief Description of the Drawings

Other characteristics and advantages of the present invention will become more evident from the description of a preferred, but not exclusive, embodiment of a device for the administration of fluid products, illustrated by way of example, but not limited to them, in the annexed drawings:

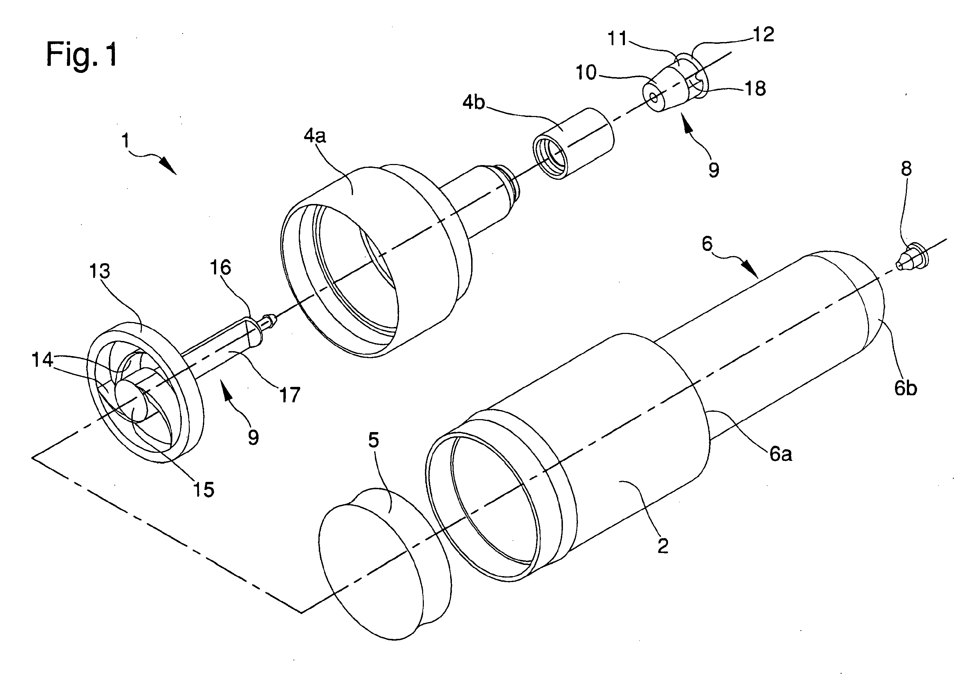

figure 1 is an exploded view of the device according to the invention;

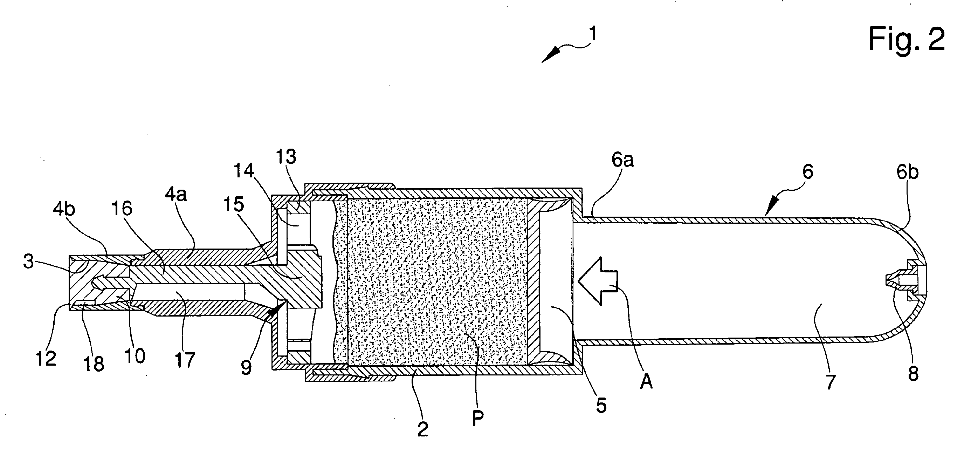

figure 2 is section view of the device according to the invention, in the initial packaging phase;

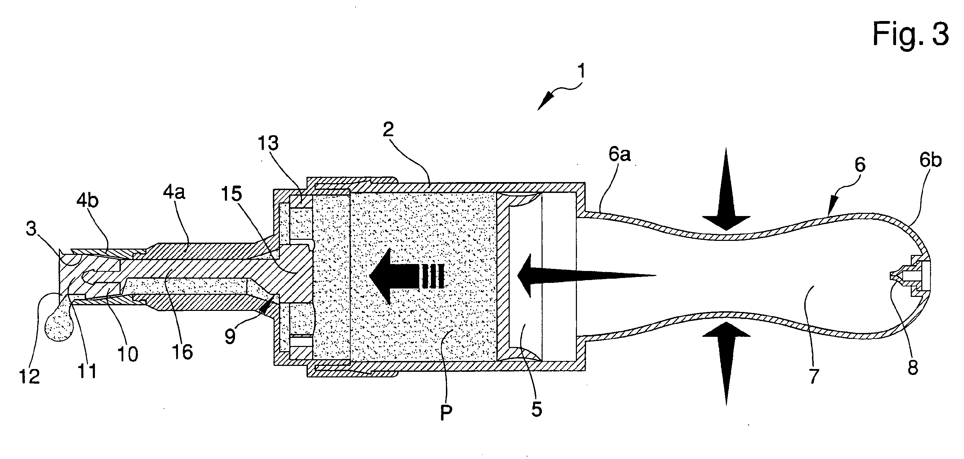

figure 3 is a section view of the device according to the invention in the dispensing phase;

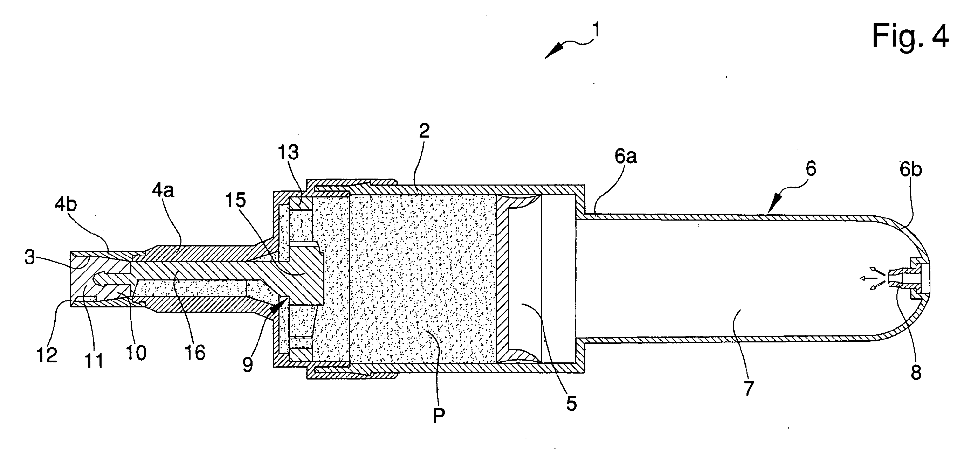

figure 4 is a section view of the device according to the invention in the phase of return to initial configuration;

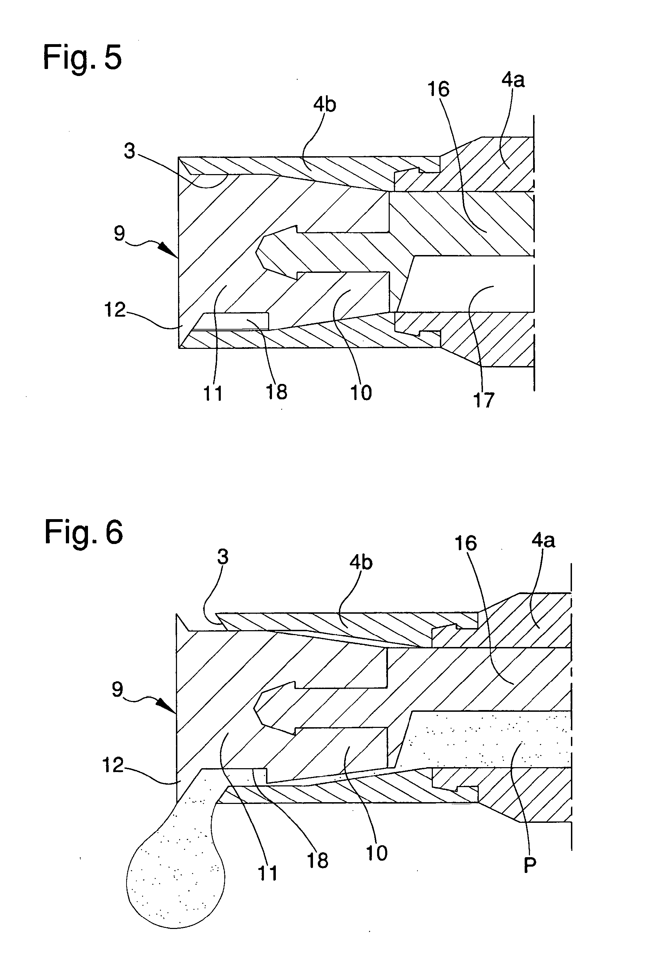

figure 5 is a section view, on an enlarged scale, of a detail of figure 2;
figure 6 is a section view, on an enlarged scale, of a detail of figure 3.

### Embodiments of the Invention

With particular reference to such figures, by 1 is globally indicated a device for the administration of fluid products.

In this respect, it is specified that, in the present treatise, by the term fluid products is not meant only liquid products but also viscous products, e.g., in paste or gel state, and powdered products, in particular very fine powders with high flowability.

The device 1 comprises a tubular body 2 for containing a fluid product P to be administered and connected to an outlet opening 3 for dispensing the fluid product P.

The tubular body 2, in particular, consists in a cylindrical section with round cross section which, at one extren 3a s associated slotted with a shaped element defining a dispensing neck 4a, 4b.

The dispensing neck 4a, 4b, is split into a first section 4a in substantially rigid material and into a second section 4b in substantially soft material, of the type of an elastomer or the like, in correspondence to which the outlet opening 3 is obtained.

Inside the cylindrical section 2, a piston 5 is fitted sealed which is axially sliding along a sliding direction A, between an initial packaging position, substantially distant with respect to the outlet opening 3, and an occurred dispensing end position wherein the piston 5 is in the proximity of the outlet opening 3.

To the tubular body 2 a deformable element 6 is connected sealed, suitable for defining an air chamber 7 to operate the piston 5, the crushing of the deformable element 6 being suitable for putting under pressure the air in the air chamber 7 to push the piston 5 along the sliding direction A.

Usefully, the deformable element 6 is substantially flexible, the release of the deformable element 6 after crushing being suitable to return the deformable element 6 to the initial configuration.

In particular, the deformable element 6 comprises a cap having a first extremity 6a associated sealed with the tubular body 2 and a second extremity 6b opposite the first extremity 6a.

In the particular embodiment of the invention shown in the illustrations, the tubular body 2 and the cap are made in a single body piece, e.g., by the injection moulding of plastic material, having a greater thickness of the plastic in correspondence to the cylindrical section 2, so as to provide sufficient rigidity, and a lesser thickness in correspondence to the deformable element 6, so as to provide the cap with the necessary flexibility.

Alternative embodiments cannot however be ruled out wherein, instead, the tubular body 2 and the cap are made in separate pieces and associable with one another during the assembly phase.

The cap has a substantially cylindrical shape, with axis parallel to the sliding direction A, and this particular embodiment favours transversal crushing by the user.

In this respect, it must be pointed out that, in the present treatise, by the expression "transversal crushing" is meant the application of a pressure on the side walls of the deformable element directed along a direction transversal to the sliding direction A.

In correspondence to the second extremity 6b, the cap has a round cap shape; alternative embodiments cannot however be ruled out wherein the conformation of the second extremity 6b is substantially square, parallelepiped and/or with flat portions, feet or the like which allow defining a supporting base able to allow the positioning of the device 1 substantially vertically.

Usefully, the deformable element 6 is associated with a valve element 8 for the unidirectional flow of air from outside towards the inside of the air chamber 7. Such valve element consists, e.g., of a blade valve arranged in correspondence to the second extremity 6b of the cap.

For this purpose, the second extremity 6b has a hole through which the blade valve 8 is fitted.

The blade valve 8 is made of elastic material (rubber or the like) and has blades orientated so as to open by effect of the flow of air from the outside towards the inside of the air chamber 7.

The device 1 also has temporary closing means 9 suitable for making it possible to temporarily close the outlet opening 3.

The temporary closing means 9 are composed, e.g., of check valve means comprising a shutter body 10, 11, 12 that can be fitted in the outlet opening 3 and is associated with an elastic element 13, 14, 15 to contrast the moving away of the shutter body 10, 11, 12 from the outlet opening 3.

The elastic element 13, 14, 15 comprises a spring of the type chosen from the list comprising: leaf springs, radial springs, helical springs.

In the particular embodiment shown in the illustrations, the elastic element 13, 14, 15 consists of a radial spring made up of a ring 13 fitted on the inner face of the shaped element defining the dispensing neck 4a, 4b, and of a series of curvilinear arms 14 extending protruding from the ring 13 towards the centre 15 of the spring.

The shutter body 10, 11, 12 is associated with the elastic element 13, 14, 15 by interposition of a connection rod 16 which, at one extremity is associated with the centre 15 of the elastic element 13, 14, 15 and which, at the opposite extremity, bears the shutter body 10, 11, 12.

The connection rod 16 is shaped to be substantially snugly fitted in the dispensing neck 4a, 4b and comprises a recess 17 to convey the fluid product P towards the outlet opening 3.

In particular, the connection rod 16 has a side surface substantially complementary to the inner surface of the dispensing neck 4a, 4b, except for the part corresponding to the recess 17, which thus defines the only access way for the fluid product P to come out.

The shutter body 10, 11, 12 comprises a substantially truncated-cone portion 10 which can be seal-coupled with a surface substantially complementary to the outlet opening 3 to define a cone-on-cone coupling.

The shutter body 10, 11, 12, also comprises a substantially cylindrical portion 11 coupled to a corresponding surface of the outlet opening 3 to define a sliding coupling.

Finally, the shutter body 10, 11, 12 ends in a widened closing edge 12 that extends from the substantially cylindrical portion 11 on the opposite side with respect to the substantially truncated-cone portion 10.

Usefully, at least one between the outer surface of the shutter body 10, 11, 12 and the inner surface of the outlet opening 3 has a groove 18 for conveying the fluid product P at outlet.

The groove 18 is arranged downstream of the above cone-on-cone coupling, wherein the expression "downstream" in this treatise must be considered with reference to the normal outlet direction of the fluid product P from the device 1. In the particular embodiment of the invention shown in the illustrations, the groove 18 is obtained on the surface of the substantially cylindrical portion 11, but other embodiments cannot be ruled out wherein, instead, this is obtained on a corresponding inner section of the dispensing neck 4a, 4b.

The recess 17 and the groove 18 are arranged substantially aligned along the outlet direction of the fluid product, by which is meant that they are orientated on the same part of the device 1 so that the fluid product P at outlet crosses first the recess 17 and immediately afterwards the groove 18, being conveyed along a preset path which allows it to be practically and easily orientated by the user and, therefore, provides greater application precision.

By virtue of the recess 17 and of the groove 18, for example, the device 1 can be used not only vertically, with the outlet opening 3 turned downwards, but also horizontally, with the outlet opening 3 arranged sideways; this is especially useful and advantageous, for example, for eye applications of the fluid product P, when an inconvenient positioning of the device 1 risks hindering the user's vision.

To protect the dispensing area when the device 1 is not in use, a cover cap can be usefully provided not shown in the illustrations, to be fitted on the dispensing neck 4a, 4b.

The operation of the present invention is the following.

The device 1 is placed on the market with the tubular body 2 filled with fluid product P and the piston 5 arranged in the initial packaging position, substantially away from the outlet opening 3 and ready to dispense the fluid product P.

At the time of use, the user applies a compression force on the cap which is directed transversally to the sliding direction A, deforming its walls and pressurising the air inside the air chamber 7.

In this phase, the blade valve 8 prevents the air under pressure from escaping from the air chamber 7.

The increase in pressure in the air chamber 7 creates a thrust force on the piston 5 which starts its movement along the sliding direction A.

The piston 5, therefore, pushes the fluid product P towards the outlet opening 3, determining the sliding of the connection rod 16 and of the shutter body 10, 11, 12 outwards.

The fluid product P, therefore, overflows through the recess 17, until it reaches the shutter body 10, 11, 12, and through the groove 18, from where it escapes outside.

Once the cap is released, the elastic return of the deformable element 6 to the initial configuration determines the suction of the air from outside through the blade valve 8 and the new filling of the air chamber 7.

The piston 5, no longer under pressure, stops pushing the fluid product P and the shutter body 10, 11, 12 returns to the closed position of the outlet opening 3 by effect of the recall force exercised by the elastic element 13,14, 15.

The dispensing of the fluid product P, therefore, is interrupted until the cap is once again crushed to allow the further forward movement of the piston 5 as far as the end-of-stroke position inside the tubular body 2, corresponding to the final dispensing-completed position.

It has in fact been ascertained how the described invention achieves the proposed objects.

In this respect, the fact is underlined that the particular solution of providing an air chamber communicating with the piston for the dispensing of the fluid product, allows handling the device according to the invention in a practical, easy and functional way even in particular uses such as, for example, applying eye-drops or the like.

The deformable element provided in the present invention in fact can be easily crushed by means of the application of a force transversal to the sliding direction of the piston, unlike traditional syringe devices wherein, instead, the thrust action is applied from outside along an axial direction.

Nor should it be forgotten that the particular solution of providing a specific shaped shutter body as in the present invention, allows, in closed configuration, ensuring the necessary seal of the device and, in dispensing configuration, to orientate and direct the flow of fluid product at outlet for easy and precise application.

## Claims

1. Device (1) for the administration of fluid products, comprising:
- at least a tubular body (2) for containing a fluid product (P) to be administered and connected to at least an outlet opening (3) for said fluid product (P),
- temporary closing means (9) of said outlet opening (3),
- at least a piston (5) which can axially slide sealed inside said tubular body (2) along a sliding direction (A),
- at least a deformable element (6) connected sealed to said tubular body (2) and defining an air chamber (7) for the operation of said piston (5), crushing said deformable element (6) being suitable for pressurising the air in said air chamber (7) to push said piston (5) along said sliding direction (A),
**characterized by** the fact that said temporary closing means (9) of said outlet opening (3) comprise check valve means comprising at least a shutter body (10, 11, 12) which can be fitted in said outlet opening (3) and associated with at least an elastic element (13, 14, 15) to contrast the moving away of said shutter body (10, 11, 12) from said outlet opening (3), at least one between the outer surface of said shutter body (10, 11, 12) and the inner surface of said outlet opening (3) comprising at least a groove (18) for conveying said fluid product (P) at outlet.

2. Device (1) according to claim 1, **characterised by** the fact that said deformable element (6) is substantially elastic and once said deformable element (6) is released, after said crushing, said deformable element (6) returns in the initial configuration.

3. Device (1) according to one or more of the preceding claims, **characterised by** the fact that said deformable element (6) comprises a cap having a first extremity (6a) connected sealed to said tubular body (2) and a second extremity (6b) opposite said first extremity (6a).

4. Device (1) according to one or more of the preceding claims, **characterised by** the fact that said deformable element (6) is associated with at least a valve element (8) for the unidirectional flow of air from outside towards the inside of said air chamber (7).

5. Device (1) according to claims 3 and 4, **characterised by** the fact that said valve element (8) is associated in correspondence to said second extremity (6b) of the cap.

6. Device (1) according to one or more of the preceding claims, **characterised by** the fact that said tubular body (2) and said deformable element (6) are made in a single body piece.

7. Device (1) according to one or more of the preceding claims, **characterised by** the fact that said elastic element (13, 14, 15) comprises at least a spring of the type chosen from the list comprising: leaf springs, radial springs, helical springs.

8. Device (1) according to one or more of the preceding claims, **characterised by** the fact that said shutter body (10, 11, 12) comprises at least a substantially truncated-cone portion (10) that can be coupled sealed with a substantially complementary surface of said outlet opening (3) to define a cone-on-cone coupling.

9. Device (1) according to claim 8, **characterised by** the fact that said groove (18) is arranged downstream of said cone-on-cone coupling.

10. Device (1) according to one or more of the preceding claims, **characterised by** the fact that said tubular body (2) is associated with at least a dispensing neck (4a, 4b) in correspondence to which said outlet opening (3) is obtained, and by the fact that said shutter body (10, 11, 12) is associated with said elastic element (13, 14, 15) by interposition of a connection rod (16) fitted in said dispensing neck (4a, 4b).

11. Device (1) according to claim 10, **characterised by** the fact that said connection rod (16) is substantially snugly fitted in said dispensing neck (4a, 4b) and comprises at least a recess (17) to convey said fluid product (P) to said outlet opening (3).

12. Device (1) according to claim 11, **characterised by** the fact that said recess (17) and said groove (18) are arranged substantially aligned along the outlet direction of said fluid product (P).

## Patentansprüche

1. Vorrichtung (1) zur Verabreichung von Fluid-Produkten, umfassend:
- zumindest einen rohrförmigen Körper (2) zur Aufnahme eines zu verabreichenden Fluid-Produktes (P), der mit zumindest einer Abgabeöffnung (3) für das Fluid-Produkt (P) verbunden wird,
- temporäre Verschlussmittel (9) der Abgabeöffnung (3), und
- zumindest einen Kolben (5), der axial abgedichtet in einer Verschieberichtung (A) in dem rohrförmigen Körper (2) gleiten kann,
- zumindest ein verformbares Element (6), das abgedichtet mit dem rohrförmigen Körper (2) verbunden ist und eine Luftkammer zur Betätigung des Kolbens (5) definiert, wobei die Luft in der Luftkammer (7) durch Stauchen des verformbaren Elementes (6) komprimiert werden kann, um den Kolben (5) entlang der Verschieberichtung (A) zu drücken,
**dadurch gekennzeichnet, dass** die temporären Verschlussmittel (9) der Abgabeöffnung (3) Rückschlagventil-Mittel umfassen, die zumindest einen Verschlusskörper (10, 11, 12) umfassen, der in der Abgabeöffnung (3) angeordnet werden und mit zumindest einem elastischen Element (13, 14, 15) zusammenwirken kann, um der Fortbewegung des Verschlusskörpers (10, 11, 12) von der Abgabeöffnung (3) entgegenzuwirken, wobei zumindest eine aus der Gruppe umfassend die Außenfläche des Verschlusskörpers (10, 11, 12) und die Innenfläche der Abgabeöffnung (3) zumindest eine Nut (18) zur Förderung des Fluid-Produkts (P) zur Abgabe umfasst.

2. Vorrichtung (1) gemäß Anspruch 1, **dadurch gekennzeichnet, dass** das verformbare Element (6) im Wesentlichen elastisch ist, und dass sobald das verformbare Element (6) losgelassen wird, das verformbare Element (6) nach der Stauchung in die ursprüngliche Konfiguration zurückkehrt.

3. Vorrichtung (1) gemäß einem oder mehreren der vorigen Ansprüche, **dadurch gekennzeichnet, dass** das verformbare Element (6) eine Abdeckung umfasst, die ein erstes Ende (6a) aufweist, das abgedichtet mit dem rohrförmigen Körper (2) verbunden ist, sowie ein zweites Ende (6b), das dem ersten Ende (6a) gegenüberliegt.

4. Vorrichtung (1) gemäß einem oder mehreren der vorigen Ansprüche, **dadurch gekennzeichnet, dass** das verformbare Element (6) mit zumindest einem Ventilelement (8) für den einseitig gerichteten Luftfluss von außen ins Innere der Luftkammer (7) zusammenwirkt.

5. Vorrichtung (1) gemäß Anspruch 3 oder 4, **dadurch gekennzeichnet, dass** das Ventilelement (8) in Bezug auf das zweite Ende (6b) der Abdeckung angeordnet ist.

6. Vorrichtung (1) gemäß einem oder mehreren der vorigen Ansprüche, **dadurch gekennzeichnet, dass** der rohrförmige Körper (2) und das verformbare Element (6) einstückig ausgebildet sind.

7. Vorrichtung (1) gemäß einem oder mehreren der vorigen Ansprüche, **dadurch gekennzeichnet, dass** das elastische Element (13, 14, 15) zumindest eine Feder der aus der folgenden Liste ausgewählten Art umfasst, wobei die Liste umfasst: Blattfedern, Radialfedern, Schraubenfedern.

8. Vorrichtung (1) gemäß einem oder mehreren der vorigen Ansprüche, **dadurch gekennzeichnet, dass** der Verschlusskörper (10, 11, 12) zumindest einen im Wesentlichen kegelstumpfförmigen Abschnitt (10) umfasst, der dichtend mit einer im Wesentlichen komplementären Oberfläche der Abgabeöffnung (3) gekoppelt werden kann, um eine Kegel-an-Kegel-Kopplung vorzugeben.

9. Vorrichtung (1) gemäß Anspruch 8, **dadurch gekennzeichnet, dass** die Nut (18) stromabwärts der Kegel-an-Kegel-Kopplung angeordnet ist.

10. Vorrichtung (1) einem oder mehreren der vorigen Ansprüche, **dadurch gekennzeichnet, dass** der rohrförmige Körper (2) mit zumindest einem Abgabestutzen (4a, 4b) zusammenwirkt, woraus sich entsprechend die Abgabeöffnung (3) ergibt, und dadurch, dass der Verschlusskörper (10, 11, 12) durch Zwischenschaltung einer in dem Abgabestutzen (4a, 4b) angeordneten Verbindungsstange (16) mit dem elastischen Element (13, 14, 15) zusammenwirkt.

11. Vorrichtung (1) gemäß Anspruch 10, **dadurch gekennzeichnet, dass** die Verbindungsstange (16) sich im Wesentlichen an den Abgabestutzen (4a, 4b) anschmiegt und zumindest eine Ausnehmung (17) umfasst, um das Fluid-Produkt (P) zur Abgabeöffnung (3) zu fördern.

12. Vorrichtung (1) gemäß Anspruch 11, **dadurch gekennzeichnet, dass** die Ausnehmung (17) und die Nut (18) im Wesentlichen zueinander ausgerichtet entlang der Abgaberichtung des Fluid-Produkts (P) angeordnet sind.

## Revendications

1. Dispositif (1) d'administration de produits fluides, comprenant :
- au moins un corps tubulaire (2) destiné à contenir un produit fluide (P) à administrer et relié à au moins une ouverture de sortie (3) pour ledit produit fluide (P),
- des moyens de fermeture temporaire (9) de ladite ouverture de sortie (3),
- au moins un piston (5) qui peut coulisser axialement et de manière étanche à l'intérieur dudit corps tubulaire (2) le long d'une direction de coulissement (A),
- au moins un élément déformable (6) relié de manière étanche audit corps tubulaire (2) et définissant une chambre à air (7) pour le fonctionnement dudit piston (5), un écrasement dudit élément déformable (6) étant approprié pour mettre sous pression l'air dans ladite chambre à air (7) afin de pousser ledit piston (5) le long de la direction de coulissement (A),
**caractérisé par le fait que** lesdits moyens de fermeture temporaire (9) de ladite ouverture de sortie (3) comprennent des moyens de clapet anti-retour comprenant au moins un corps d'obturation (10, 11, 12) qui peut être ajusté dans ladite ouverture de sortie (3) et associé à au moins un élément élastique (13, 14, 15) pour s'opposer au mouvement d'éloignement dudit corps d'obturation (10, 11, 12) par rapport à ladite ouverture de sortie (3), au moins l'une parmi la surface externe dudit corps d'obturation (10, 11, 12) et la surface interne de ladite ouverture de sortie (3) comprenant au moins une rainure (18) destinée à acheminer ledit produit fluide (P) vers la sortie.

2. Dispositif (1) selon la revendication 1, **caractérisé par le fait que** ledit élément déformable (6) est sensiblement élastique, et en ce que, une fois que ledit élément déformable (6) est libéré, après ledit écrasement, ledit élément déformable (6) revient dans sa configuration initiale.

3. Dispositif (1) selon une ou plusieurs des revendications précédentes, **caractérisé par le fait que** ledit élément déformable (6) comprend un capuchon ayant une première extrémité (6a) reliée de manière étanche audit corps tubulaire (2) et une deuxième extrémité (6b) opposée à ladite première extrémité (6a).

4. Dispositif (1) selon une ou plusieurs des revendications précédentes, **caractérisé par le fait que** ledit élément déformable (6) est associé à au moins un élément de vanne (8) pour l'écoulement unidirectionnel de l'air de l'extérieur vers l'intérieur de ladite chambre à air (7).

5. Dispositif (1) selon les revendications 3 et 4, **caractérisé par le fait que** ledit élément de vanne (8) est associé en correspondance avec ladite deuxième extrémité (6b) du capuchon.

6. Dispositif (1) selon une ou plusieurs des revendications précédentes, **caractérisé par le fait que** ledit corps tubulaire (2) et ledit élément déformable (6) sont réalisés en un unique élément de corps.

7. Dispositif (1) selon une ou plusieurs des revendications précédentes, **caractérisé par le fait que** ledit élément élastique (13, 14, 15) comprend au moins un ressort du type choisi dans la liste comprenant : des ressorts à lames, des ressorts radiaux, des ressorts hélicoïdaux.

8. Dispositif (1) selon une ou plusieurs des revendications précédentes, **caractérisé par le fait que** ledit corps d'obturation (10, 11, 12) comprend au moins une portion sensiblement tronconique (10) qui peut être couplée de manière étanche à une surface sensiblement complémentaire de ladite ouverture de sortie (3) afin de définir un couplage du type cône sur cône.

9. Dispositif (1) selon la revendication 8, **caractérisé par le fait que** ladite rainure (18) est disposée en aval dudit couplage du type cône sur cône.

10. Dispositif (1) selon une ou plusieurs des revendications précédentes, **caractérisé par le fait que** ledit corps tubulaire (2) est associé à au moins un col de distribution (4a, 4b), en correspondance avec lequel ladite ouverture de sortie (3) est obtenue, et **par le fait que** ledit corps d'obturation (10, 11, 12) est associé audit élément élastique (13, 14, 15) au moyen de l'interposition d'une tige de liaison (16) ajustée dans ledit col de distribution (4a, 4b).

11. Dispositif (1) selon la revendication 10, **caractérisé par le fait que** ladite tige de liaison (16) est sensiblement en ajustement serré dans ledit col de distribution (4a, 4b) et comprend au moins un évidement (17) destiné à acheminer ledit produit fluide (P) vers ladite ouverture de sortie (3).

12. Dispositif (1) selon la revendication 11, **caractérisé par le fait que** ledit évidement (17) et ladite rainure (18) sont disposés sensiblement en alignement le long de la direction de sortie dudit produit fluide (P).
